# EUROPEAN PATENT APPLICATION

(11) **EP 4 170 034 A1**
(43) Date of publication of application: **26.04.2023**
(21) Application number: 21825386.2
(22) Date of filing: 16.06.2021
(51) Int. Cl.: C12N 15/54, C12N 9/10, A23L 33/195

(54) **NOVEL TRANSGLUTAMINASE**

(30) Priority: 18.06.2020 JP 2020105132
(71) Applicant: Amano Enzyme Inc., Nagoya-shi Aichi 460-8630 (JP); The University of Tokyo, Bunkyo-ku, Tokyo 113-8654 (JP)
(72) Inventor: MARUYAMA, Jun-ichi, Tokyo 113-8654 (JP); MAMUN, Md. Abdulla Al, Tokyo 113-8654 (JP); KATAYAMA, Takuya, Tokyo 113-8654 (JP); YAMAGUCHI, Shotaro, Kakamigahara-shi, Gifu 509-0109 (JP)
(74) Representative: Müller-Boré & Partner Patentanwälte PartG mbB
(86) International application number: PCT/JP2021/022918
(87) International publication number: WO 2021/256518

(57) **Abstract**

The present invention addresses the problem of providing a novel transglutaminase that is useful for food or medical applications. The present invention provides: a transglutaminase having an amino acid sequence that has an identity of 90% or more with respect to an amino acid sequence of SEQ ID NO: 1 or 2; and an enzyme preparation containing said transglutaminase as an active ingredient. The enzyme preparation is particularly useful for food production and medical applications.

## Description

### TECHNICAL FIELD

The present invention relates to a transglutaminase. Specifically, the present invention relates to a novel transglutaminase derived from koji mold, applications thereof, and the like.

### BACKGROUND ART

Transglutaminases are enzymes that catalyze an acyl transfer reaction of a γ-carboxylamide group of a glutamine residue in a peptide chain, and when an ε-amino group of a lysine residue in a protein acts as an acyl receptor, an ε-(γ-Gln)-Lys crosslinking bond is formed in or between protein molecules. Transglutaminases generally play important roles in a range of physiological functions such as cell differentiation, cell signaling, blood coagulation, and wound healing, and abnormalities in regulation of transglutaminase functions have been associated with autoimmune diseases such as neurodegenerative diseases, celiac disease, cancer, and tissue fibrosis. While studies of transglutaminase functions have been conducted in bacteria, yeast, plants, invertebrates, amphibians, fishes, and birds, transglutaminase functions of multicellular fungi have not been elucidated so far.

In addition, since a protein or a peptide can be modified by utilizing an action of a transglutaminase, a transglutaminase derived from the genus Streptomyces (see, for example, Patent Document 1) has been used for binding of meat, and production of sausages, bean curds, breads, and noodles. In addition, use of a transglutaminase has been studied not only in the food field but also in the fiber field, the medical field, the cosmetic field, and the like. With such expanded use, attempts have been made to improve properties (heat resistance, specific activity, substrate specificity, stability, and the like) of a transglutaminase (see, for example, Patent Documents 2 to 4 and Non-Patent Documents 1 to 4).

### PRIOR ART DOCUMENTS

### PATENT DOCUMENTS

Patent Document 1: Japanese Patent Laid-open Publication No. H04-108381
Patent Document 2: Japanese Patent Laid-open Publication No. 2002-253272
Patent Document 3: Japanese Patent Laid-open Publication No. 2008-194004
Patent Document 4: International Publication No. WO 2019/107288 A

### NON-PATENT DOCUMENTS

Non-Patent Document 1: Marx CK et al., J. Biotechnol. 2008 Sep 10; 136(3-4): 156-62.
Non-Patent Document 2: Tagami U et al., Protein Eng Des Sel. 2009 Dec; 22(12): 747-752.
Non-Patent Document 3: Yokoyama K et al., Appl Microbiol Biotechnol (2010) 87: 2087-2096.
Non-Patent Document 4: Buettner K et al., Amino Acids. 2012 Feb; 42(2-3): 987-96.

### SUMMARY OF THE INVENTION

### PROBLEMS TO BE SOLVED BY THE INVENTION

As described above, a transglutaminase has been used in various fields, but there is still a high need for a transglutaminase suitable for applications requiring high safety, such as food and medical fields. Therefore, an object of the present invention is to provide a novel transglutaminase that can also be used in food or medical applications, and applications thereof.

### MEANS FOR SOLVING THE PROBLEM

As a result of intensive studies to solve the above problems, the present inventors have found a novel transglutaminase by screening from a gene having an unknown function present specifically in a filamentous fungus. Genome information of Aspergillus oryzae, which is a filamentous fungus, has been analyzed, but no protein having transglutaminase activity has been found. The present inventors have successfully identified a transglutaminase derived from Aspergillus oryzae by detecting transglutaminase activity using a new method called a hypotonic shock experiment.

The present invention provides inventions of the following embodiments.
[1] A transglutaminase consisting of a protein represented by any one of (a) to (c):
   (a) a protein containing an amino acid sequence set forth in SEQ ID NO: 1 or 2;
   (b) a protein containing an amino acid sequence in which one or a few amino acids are substituted, added, inserted, or deleted in an amino acid sequence set forth in SEQ ID NO: 1 or 2, and having activity of catalyzing an acyl transfer reaction of a γ-carboxylamide group of a glutamine residue in a peptide chain; and
   (c) a protein containing an amino acid sequence having sequence identity of 90% or more to an amino acid sequence set forth in SEQ ID NO: 1 or 2, and having activity of catalyzing an acyl transfer reaction of a γ-carboxylamide group of a glutamine residue in a peptide chain.
[2] The transglutaminase according to [1], which is derived from Aspergillus oryzae.
[3] A DNA encoding the transglutaminase according to [1[ or [2].
[4] The DNA according to [3], having a base sequence having sequence identity of 90% or more to a base sequence of SEQ ID NO: 4.
[5] An enzyme preparation containing the transglutaminase according to [1] or [2] as an active ingredient.
[6] The enzyme preparation according to [5], which is used for crosslinking a protein or a peptide.
[7] The enzyme preparation according to [5], which is for production of a food.
[8] The enzyme preparation according to [5], which is for medical use or industrial use.
[9] A method for performing an acyl transfer reaction on a γ-carboxylamide group of a glutamine residue in a protein or a peptide, the method including allowing a protein represented by any one of (a) to (c) to act on a protein or a peptide as a substrate:
   (a) a protein containing an amino acid sequence set forth in SEQ ID NO: 1 or 2;
   (b) a protein containing an amino acid sequence in which one or a few amino acids are substituted, added, inserted, or deleted in an amino acid sequence set forth in SEQ ID NO: 1 or 2, and having activity of catalyzing an acyl transfer reaction of a γ-carboxylamide group of a glutamine residue in a peptide chain; and
   (c) a protein containing an amino acid sequence having sequence identity of 90% or more to an amino acid sequence set forth in SEQ ID NO: 1 or 2, and having activity of catalyzing an acyl transfer reaction of a γ-carboxylamide group of a glutamine residue in a peptide chain.
[10] The acyl transfer method according to [9], which is performed for crosslinking a protein or a peptide.
[11] A method for producing a food, the method including allowing a protein represented by any one of (a) to (c) to act on a food material containing a protein or a peptide:
   (a) a protein containing an amino acid sequence set forth in SEQ ID NO: 1 or 2;
   (b) a protein containing an amino acid sequence in which one or a few amino acids are substituted, added, inserted, or deleted in an amino acid sequence set forth in SEQ ID NO: 1 or 2, and having activity of catalyzing an acyl transfer reaction of a γ-carboxylamide group of a glutamine residue in a peptide chain; and
   (c) a protein containing an amino acid sequence having sequence identity of 90% or more to an amino acid sequence set forth in SEQ ID NO: 1 or 2, and having activity of catalyzing an acyl transfer reaction of a γ-carboxylamide group of a glutamine residue in a peptide chain.
[12] Use of a protein represented by any one of (a) to (c) for performing an acyl transfer reaction on a γ-carboxylamide group of a glutamine residue in a protein or a peptide:
   (a) a protein containing an amino acid sequence set forth in SEQ ID NO: 1 or 2;
   (b) a protein containing an amino acid sequence in which one or a few amino acids are substituted, added, inserted, or deleted in an amino acid sequence set forth in SEQ ID NO: 1 or 2, and having activity of catalyzing an acyl transfer reaction of a γ-carboxylamide group of a glutamine residue in a peptide chain; and
   (c) a protein containing an amino acid sequence having sequence identity of 90% or more to an amino acid sequence set forth in SEQ ID NO: 1 or 2, and having activity of catalyzing an acyl transfer reaction of a γ-carboxylamide group of a glutamine residue in a peptide chain.
[13] The use according to [12], which is for crosslinking a protein or a peptide.

### BRIEF DESCRIPTION OF THE DRAWINGS

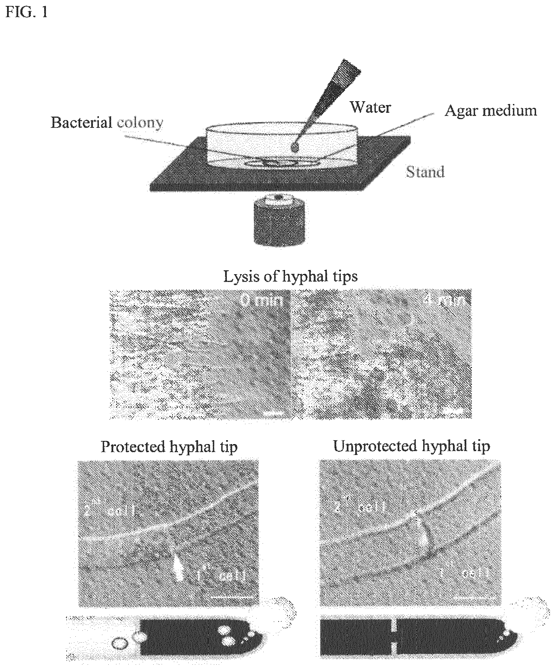

Fig. 1 shows an outline of a hypotonic shock experiment. A function of preventing adjacent cells from propagation of lysis at the time of hyphal damage is evaluated. After culturing in a DPY agar medium for 18 hours, water is added to induce lysis of hyphal tips. Thirty hyphae showing lysis of hyphal tips are randomly selected and observed with a differential interference contrast microscope to count hyphae that prevented propagation of lysis.

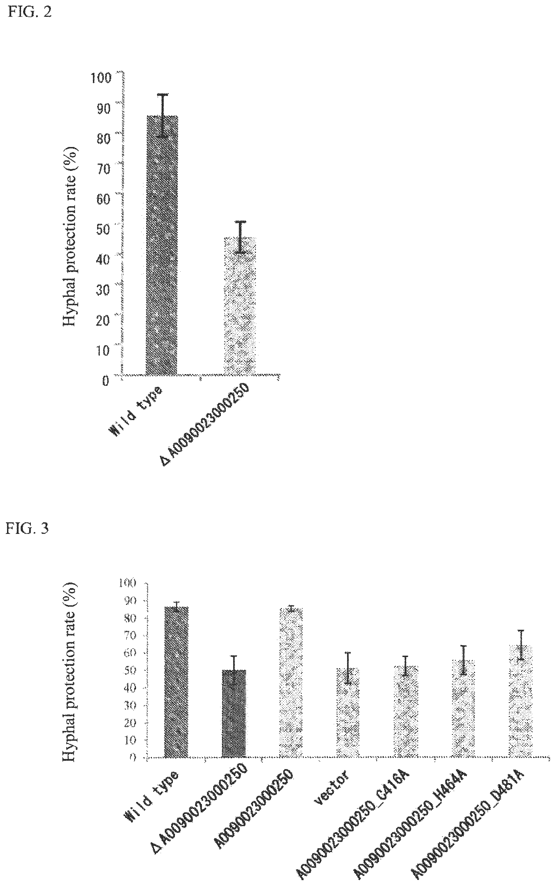

Fig. 2 shows results of a hypotonic shock experiment. Specifically, a hyphal protection rate in a wild-type strain (Wild type; NSP1D1 strain) and an AO090023000250-disrupted strain (ΔAO090023000250) is shown.
Fig. 3 shows results of a hypotonic shock experiment. Specifically, a hyphal protection rate in strains in which amino acids at an active center are substituted of AO090023000250 is shown. In the figure, "Wild type" is a wild-type strain (NSP1D1 strain), "ΔAO090023000250" is an AO090023000250-disrupted strain, "AO090023000250" is a strain obtained by introducing AO090023000250 into the AO090023000250-disrupted strain, "vector" is a strain obtained by introducing an empty vector having no insert DNA into the AO090023000250-disrupted strain, "ΔAO090023000250_C417A" is a strain obtained by introducing AO090023000250 encoding a C417A substitution, "ΔAO090023000250_H464A" is a strain obtained by introducing AO090023000250 encoding an H464A substitution, and "ΔAO090023000250_D480A" is a strain obtained by introducing AO090023000250 encoding a D480A substitution.

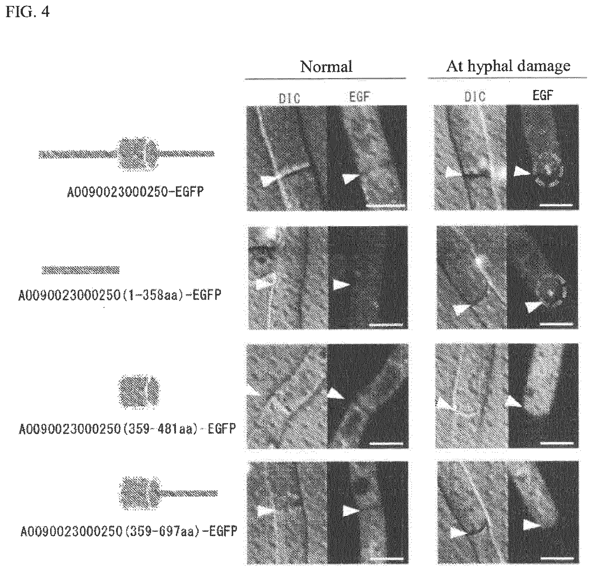

Fig. 4 shows observation results of intracellular localization by a fluorescence microscope. Specifically, results of intracellular localization of partially deleted form of AO090023000250 are shown. The arrowhead indicates a septum, and accumulation in a septal pore is indicated by the dotted circle. Scale bar: 5 µm. In Fig. 4, "AO090023000250-EGFP" indicates a strain obtained by introducing a fusion protein of full-length AO090023000250 and EGFP into an AO090023000250-disrupted strain; "AO090023000250(1-358aa)-EGFP" indicates a strain obtained by introducing a fusion protein of AO090023000250 in which a C-terminal region (region from positions 359 to 697 of an amino acid sequence set forth in SEQ ID NO: 1) is deleted and EGFP into an AO090023000250-disrupted strain; "AO090023000250(359-481aa)-EGFP" indicates a strain obtained by introducing a fusion protein of AO090023000250 in which a C-terminal region (region from positions 482 to 697 of the amino acid sequence set forth in SEQ ID NO: 1) and an N-terminal region (region from positions 1 to 358 of the amino acid sequence set forth in SEQ ID NO: 1) are deleted and EGFP into an AO090023000250-disrupted strain; "AU090023000250(359-697aa)-EGFP" indicates a strain obtained by introducing a fusion protein of AO090023000250 in which an N-terminal region (region from positions 1 to position 358 of the amino acid sequence set forth in SEQ ID NO: 1) is deleted and EGFP into an AO090023000250-disrupted strain; "DIC" indicates a differential interference contrast micrograph; "EGF" indicates a fluorescence micrograph of observed green fluorescence by EGEP; "Normal" indicates before a hypotonic shock experiment; and "At hyphal damage" indicates after the hypotonic shock experiment.

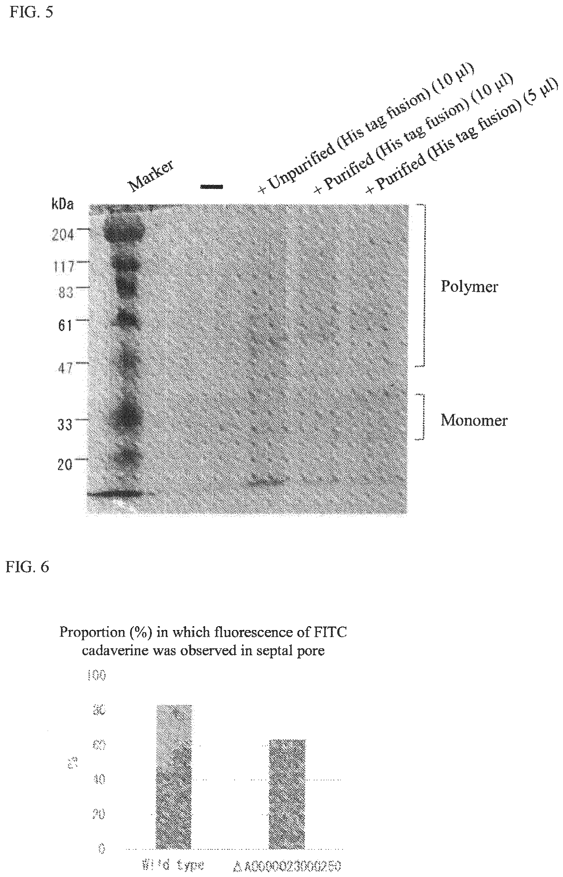

Fig. 5 shows results of a polymerization assay of casein (SDS-PAGE). Results of, from left, a molecular weight marker, enzyme-untreated casein, unpurified 3 × HA-6His tag fusion transglutaminase (10 µL)-treated casein, purified 3 × HA-6His tag fusion transglutaminase (10 µL)-treated casein, and purified 3 × HA-6His tag fusion transglutaminase (5 µL)-treated casein.
Fig. 6 shows an evaluation of transglutaminase activity using 5-FITC cadaverine. Specifically, in an AO090023000250-disrupted strain and a wild-type strain (NSP1D1 strain), a proportion of hyphae in which fluorescence is observed in a septal pore is shown.

### EMBODIMENTS OF THE INVENTION

The term "transglutaminase derived from Aspergillus oryzae" refers to a transglutaminase whose origin is Aspergillus oryzae, and includes a transglutaminase produced by Aspergillus oryzae or a transglutaminase expressed in another microorganism or the like using genetic information of the transglutaminase.

### 1. Transglutaminase

A first aspect of the present invention relates to a transglutaminase (hereinafter also referred to as "present enzyme"). The transglutaminase of the present invention is particularly useful for food or medical applications.

One embodiment of the transglutaminase of the present invention consists of (a) a protein containing an amino acid sequence set forth in SEQ ID NO: 1 or 2.

The amino acid sequence set forth in SEQ ID NO: 1 is a full amino acid sequence length of a transglutaminase derived from Aspergillus oryzae that the present inventors have successfully identified. Studies by the present inventors have revealed that a protein derived from Aspergillus oryzae consisting of the amino acid sequence set forth in SEQ ID NO: 1 exhibits transglutaminase activity, that is, functions as a transglutaminase. In addition, as shown in Examples described below, the protein exhibited a property of being able to prevent lysis of hyphal tip cells due to a hypotonic shock from propagating to adjacent cells. In addition, it has been confirmed that the protein catalyzes protein crosslinking by an experiment using a casein protein, and that the protein exhibits transglutaminase activity by an experiment using 5-FITC cadaverine.

In addition, as a result of studies by the present inventors, it was revealed that an N-terminal region at positions 1 to 358 of the amino acid sequence of SEQ ID NO: 1 is a sequence involved in localization (localization sequence), and it was predicted that the N-terminal region is not involved in transglutaminase activity. Therefore, it can be rationally predicted that a protein consisting of a partial sequence obtained by removing the localization sequence from the amino acid sequence of SEQ ID NO: 1 (SEQ ID NO: 2; amino acid sequence at positions 359 to 697 in SEQ ID NO: 1) also functions as a transglutaminase. Therefore, as one embodiment of the present invention, a protein consisting of an amino acid sequence set forth in SEQ ID NO: 2 can also be used as a transglutaminase.

Another embodiment of the transglutaminase of the present invention consists of an amino acid sequence equivalent to an amino acid sequence set forth in SEQ ID NO: 1 or 2. The term "equivalent amino acid sequence" as used herein refers to an amino acid sequence that is partially different from a reference amino acid sequence (amino acid sequence set forth in SEQ ID NO: 1 or 2), but the difference does not substantially affect a function of the protein (here, transglutaminase activity). Therefore, a protein having an amino acid sequence equivalent to the amino acid sequence set forth in SEQ ID NO: 1 or 2 catalyzes a transglutaminase reaction. The degree of activity is not particularly limited as long as a function as a transglutaminase can be exhibited. However, the activity is preferably about the same as or higher than that of a protein consisting of the reference amino acid sequence (having the amino acid sequence set forth in SEQ ID NO: 1 or 2).

The amino acid sequence equivalent to the amino acid sequence set forth in SEQ ID NO: 1 or 2 occurs by, for example, deletion or substitution of one or several amino acids among amino acids constituting the amino acid sequence set forth in SEQ ID NO: 1 or 2, addition or insertion of one or a few amino acids in the amino acid sequence set forth in SEQ ID NO: 1 or 2, or any combination thereof. In addition, examples of the amino acid sequence equivalent to the amino acid sequence set forth in SEQ ID NO: 1 or 2 include an amino acid sequence in which the number of amino acids different from amino acids of the amino acid sequence set forth in SEQ ID NO: 1 or 2 corresponds to less than about 10%. Therefore, specific examples of another embodiment of the transglutaminase of the present invention include those consisting of a protein represented by (b) or (c).
(b) A protein containing an amino acid sequence in which one or a few amino acids are substituted, added, inserted, and/or deleted in an amino acid sequence set forth in SEQ ID NO: 1 or 2, and having activity of catalyzing an acyl transfer reaction of a γ-carboxylamide group of a glutamine residue in a peptide chain.
(c) A protein containing an amino acid sequence having sequence identity of 90% or more to an amino acid sequence set forth in SEQ ID NO: 1 or 2, and having activity of catalyzing an acyl transfer reaction of a γ-carboxylamide group of a glutamine residue in a peptide chain.

In the proteins of (b) and (c), the position of an amino acid that is different from an amino acid of the amino acid sequence set forth in SEQ ID NO: 1 or 2 is not particularly limited as long as the proteins have activity of catalyzing an acyl transfer reaction of a γ-carboxylamide group of a glutamine residue in a peptide chain. Cysteine at position 417, histidine at position 464, and aspartic acid at position 480 in SEQ ID NO: 1, and cysteine at position 59, histidine at position 106, and aspartic acid at position 122 in SEQ ID NO: 2 are presumed to be active centers, and thus it is desirable that there is no mutation (substitution, deletion, and insertion) at these sites in the proteins of (b) and (c). In addition, since regions at positions 359 to 481 in SEQ ID NO: 1 and regions at positions 1 to 123 in SEQ ID NO: 2 are presumed to be active domains of a transglutaminase, as an example of the proteins of (b) and (c), there is no mutation (substitution, deletion, and insertion) in these regions.

Suitable examples of the proteins of (b) and (c) include those in which amino acid residues that are not essential for transglutaminase activity are subjected to conservative amino acid substitution in the amino acid sequence set forth in SEQ ID NO: 1 or 2. The term "conservative amino acid substitution" as used herein refers to substitution of a certain amino acid residue with an amino acid residue having a side chain having similar properties. Amino acid residues are classified into several families according to their side chains, such as basic side chains (e.g., lysine, arginine, and histidine), acidic side chains (e.g., aspartic acid and glutamic acid), uncharged polar side chains (e.g., glycine, asparagine, glutamine, serine, threonine, tyrosine, and cysteine), nonpolar side chains (e.g., alanine, valine, leucine, isoleucine, proline, phenylalanine, methionine, and tryptophan), β-branched side chains (e.g., threonine, valine, and isoleucine), and aromatic side chains (e.g., tyrosine, phenylalanine, tryptophan, and histidine). The conservative amino acid substitution is preferably substitution between amino acid residues within the same family.

In the protein of (b), the number of inserted mutations (substitutions, additions, insertions, and/or deletions) in the amino acid sequence set forth in SEQ ID NO: 1 or 2 is not particularly limited, but, for example, deletion and/or substitution of 1 to 40 (preferably 1 to 30, more preferably 1 to 10, still more preferably 1 to 7, still more preferably 1 to 5, and still more preferably 1 to 3) amino acids in the amino acid sequence set forth in SEQ ID NO: 1 or 2; addition and/or insertion of 1 to 40 (preferably 1 to 30, more preferably 1 to 10, still more preferably 1 to 7, still more preferably 1 to 5, and still more preferably 1 to 3) amino acids in the amino acid sequence set forth in SEQ ID NO: 1 or 2; and those in which a mutation (change) occurs in the amino acid sequence by a combination thereof are exemplified.

Examples of the protein of (c) include those having sequence identity of preferably about 92% or more, more preferably about 94% or more, still more preferably about 96% or more, still more preferably about 98% or more, and most preferably about 99% or more to the amino acid sequence set forth in SEQ ID NO: 1 or 2.

In the present invention, identity (%) between two amino acid sequences or two base sequences (hereinafter "two sequences" are used as a term including these) can be determined, for example, by the following procedure. First, two sequences are arranged so that an optimal comparison can be made (for example, a gap may be introduced into a first sequence to optimize alignment with a second sequence). When a molecule (amino acid residue or nucleotide) at a specific position in the first sequence is the same as a molecule at the corresponding position in the second sequence, it can be said that the molecules at that position are the same. Identity between two sequences is a function of the number of identical positions common to the two sequences (i.e., identity (%) = number of identical positions/total number of positions × 100), and preferably, the number and size of gaps required for optimization of alignment are also taken into consideration.

Comparison of two sequences and determination of identity can be realized using a mathematical algorithm. Specific examples of a mathematical algorithm available for comparison of sequences include the algorithm described in Karlin and Altschul (1993) Proc. Natl. Acad. Sci. USA 90: 5873-77. Such an algorithm is incorporated into the NBLAST program and the XBLAST program (version 2.0) described in Altschul et al. (1990) J. Mol. Biol. 215: 403-10. In order to obtain an equivalent nucleotide sequence, for example, BLAST nucleotide search may be performed using the NBLAST program with score = 100 and wordlength = 12. In order to obtain an equivalent amino acid sequence, for example, BLAST polypeptide search may be performed using the XBLAST program with score = 50 and wordlength = 3. To obtain gap alignments for comparison, Gapped BLAST is available as described in Altschul et al. (1997) Amino Acids Research 25(17): 3389-3402. When BLAST and Gapped BLAST are utilized, default parameters of the corresponding programs (e.g., XBLAST and NBLAST) can be used. For details, refer to http://www.ncbi.nlm.nih.gov. Examples of other mathematical algorithms available for comparison of sequences include the algorithm described in Myers and Miller (1988) Comput Appl Biosci. 4: 11-17. Such an algorithm is incorporated into, for example, an ALIGN program available in the GENE STREAM network server (Institute of Human Genetics, Montpellier, France) or the ISREC server. When the ALIGN program is utilized for comparison of amino acid sequences, for example, a PAM120 weight residue table can be used, and gap length penalty = 12 and gap penalty = 4 can be set.

The identity between two amino acid sequences can be determined using the Blosum 62 matrix using the GAP program in the EMBOSS package with gap weight = 10 and gap length weight = 2. In addition, identity between two base sequences can be determined using the GAP program in the EMBOSS package (available in http://emboss.open-bio.org/) with gap weight = 50 and gap length weight = 3.

In the proteins of (b) and (c), "activity of catalyzing an acyl transfer reaction of a γ-carboxylamide group of a glutamine residue in a peptide chain" can be measured according to the following method.

First, 5 to 10 µL of a solution containing a protein to be measured is mixed with 10 µL of a 10 wt% N,N-dimethylated casein solution and 35 µL of a reaction buffer (50 mM HEPES (pH 7.5), 500 mM NaCl, 10 mM CaCl2, 1 mM DTT), and the mixture is reacted at 30°C for 2 hours. Next, the solution after the reaction is migrated by SDS-PAGE, and the presence or absence of polymerized casein is confirmed by Coomassie blue staining. When polymerized casein is observed, the protein to be measured is determined to have "activity of catalyzing an acyl transfer reaction of a γ-carboxylamide group of a glutamine residue in a peptide chain".

Suitable examples of the proteins of (b) and (c) include those having transglutaminase equivalent to that of an unmodified protein (i.e., a protein containing the amino acid sequence set forth in SEQ ID NO: 1 or 2). Specific examples thereof include those in which a relative value of transglutaminase activity of the proteins of (b) and (c) is 50% or more, preferably 50 to 200%, more preferably 70 to 150%, still more preferably 80 to 120%, and particularly preferably 90 to 110% when transglutaminase activity of the protein of (a) is defined as 100%. Here, the relative value of transglutaminase activity is a value calculated with transglutaminase activity of a protein containing the amino acid sequence set forth in SEQ ID NO: 1 as 100% when the proteins of (b) and (c) have an amino acid sequence obtained by modifying the amino acid sequence set forth in SEQ ID NO: 1, and is a value calculated with transglutaminase activity of a protein containing the amino acid sequence set forth in SEQ ID NO: 2 as 100% when the proteins of (b) and (c) have an amino acid sequence obtained by modifying the amino acid sequence set forth in SEQ ID NO: 2. In addition, the transglutaminase activity is a value measured by the activity measurement method shown below.

### <'Γransglutaminase activity measurement method>

A protein to be measured is diluted with 200 mM Tris-HCl pH 6.0 to an appropriate concentration (sample solution). To 10 µL of the sample solution, 100 µL of a substrate solution (R-1) is added and mixed, and then the mixture is reacted at 37°C for 10 minutes. After 100 µL of a coloring solution (R-2) is added to stop the reaction and form an Fe complex, absorbance at 525 nm is measured. As a control, absorbance of a solution subjected to a reaction in the same manner using an enzyme solution previously heat-inactivated is measured, and a difference in absorbance from the sample solution is determined. Separately, a calibration curve is prepared using L-glutamic acid-γ-monohydroxamic acid instead of the enzyme solution, and an amount of hydroxamic acid produced from the difference in absorbance is determined. Enzyme activity for producing 1 µmol of hydroxamic acid per minute is defined as 1 unit (1 U).

### (Substrate solution (R-1))

2.42 g of 2-amino-2-hydroxymethyl-1.3-propanediol, 0.70 g of hydroxyammonium hydrochloride, 0.31 g of reduced glutathione, and 1.01 g of Z-Gln-Gly (benzyloxycarbonyl-L-glutaminylglycine) are dissolved in distilled water to make a total amount of 100 mL (pH 6.0).

### (Substrate solution (R-2))

30 mL of a 3 M hydrochloric acid solution, 30 mL of a 12% trichloroacetic acid solution, and 30 mL of a 5% iron(III) chloride solution are mixed.

The transglutaminase of the present invention may be, for example, part of a fusion protein that is fused with another protein. Examples of a sequence added in the fusion protein include a sequence useful for purification such as a multiple histidine residue, and an additional sequence that ensures stability during recombinant production.

The transglutaminase of the present invention can be obtained by culturing a microorganism that produces the transglutaminase (transglutaminase-producing strain), for example, Aspergillus oryzae. The transglutaminase-producing strain may be a wild-type strain or a mutant strain (for example, a mutant strain is obtained by ultraviolet irradiation). Specific examples of the transglutaminase-producing strain include an Aspergillus oryzae RIB40 strain.

The transglutaminase of the present invention can be prepared from a culture solution and/or a cell body of a microorganism that produces the transglutaminase of the present invention. The culture conditions and the culture method are not particularly limited as long as the transglutaminase of the present invention is produced. In other words, provided that the transglutaminase of the present invention is produced, a method and culture conditions suitable for culturing a microorganism to be used can be appropriately set. The culture method may be either liquid culture or solid culture, but liquid culture is preferably used. By taking liquid culture as an example, the culture conditions will be described.

The medium is not particularly limited as long as the used microorganism can grow in the medium. For example, those to which a carbon source such as glucose, sucrose, gentiobiose, soluble starch, glycerin, dextrin, molasses, or organic acid; further a nitrogen source such as ammonium sulfate, ammonium carbonate, ammonium phosphate, or ammonium acetate, or such as peptone, yeast extract, corn steep liquor, casein hydrolysate, bran, or meat extract; and further an inorganic salt such as a potassium salt, a magnesium salt, a sodium salt, a phosphate salt, a manganese salt, an iron salt, or a zinc salt are added can be used. In order to promote growth of a microorganism to be used, vitamins, amino acids, and the like may be added to the medium. A pH of the medium is adjusted to, for example, about 3 to 8, preferably about 4 to 7, and culture is performed under aerobic conditions at a culture temperature of usually about 20 to 40°C, preferably about 25 to 35°C, for about 1 to 20 days, preferably about 3 to 10 days. As the culture method, for example, a shaking culture method and an aerobic deep culture method using a jar fermenter can be used.

After culture under the above conditions, the target enzyme is recovered from the culture solution or the cell body. In the case of recovering the enzyme from the culture solution, the present enzyme can be obtained, for example, by filtering and centrifuging the culture supernatant to remove insoluble matters, followed by separation and purification by appropriately combining concentration with an ultrafiltration membrane, salting-out such as ammonium sulfate precipitation, dialysis, and various types of chromatography such as ion exchange resin, and the like. On the other hand, when the enzyme is recovered from the cell body, the present enzyme can be obtained, for example, by disrupting the cell body by pressurization treatment, ultrasonic treatment, or the like, followed by separation and purification in the same manner as described above. The above series of steps (disruption, separation, and purification of the cell body) may be performed after the cell body is previously recovered from the culture solution by filtration, centrifugation, or the like.

The transglutaminase of the present invention can also be easily prepared by a genetic engineering technique. For example, the transglutaminase can be prepared by transforming an appropriate host cell (e.g., Escherichia coli) with a DNA encoding the present enzyme and recovering a protein expressed in the transformant. The recovered protein is appropriately purified according to the purpose. As described above, various modifications are possible if a target enzyme is obtained as a recombinant protein. For example, when the DNA encoding the transglutaminase of the present invention and other appropriate DNAs are inserted into the same vector and a recombinant protein is produced using the vector, the transglutaminase of the present invention containing a recombinant protein to which any peptide or protein is linked can be obtained. Furthermore, modification may be performed such that addition of sugar chains and/or lipids or N-terminal or C-terminal processing occurs. By the modification as described above, extraction of a recombinant protein, simplification of purification, addition of a biological function, or the like is possible.

Usually, the transglutaminase of the present invention can be produced using an appropriate host-vector system as described above, but the transglutaminase of the present invention may be produced using a cell-free synthesis system. Here, the term "cell-free synthesis system (cell-free transcription system, cell-free transcription/translation system)" refers to synthesizing, from a nucleic acid (DNA or mRNA) as a template, mRNA or protein encoded thereby in vitro using ribosomes, transcription/translation factors, or the like derived from living cells (or obtained by a genetic engineering technique) instead of using living cells. In the cell-free synthesis system, generally, a cell extract obtained by purifying a cell-disrupted liquid as necessary is used. The cell extract generally contains ribosomes required for protein synthesis, various factors such as initiation factors, and various enzymes such as tRNA. When protein synthesis is performed, various amino acids, energy sources such as ATP and GTP, and other substances necessary for protein synthesis, such as creatine phosphate, are added to the cell extract. Of course, ribosomes, various factors, and/or various enzymes separately prepared may be supplemented as necessary during protein synthesis.

Development of a transcription/translation system in which each molecule (factor) necessary for protein synthesis is reconstituted has also been reported (Shimizu, Y. et al.: Nature Biotech., 19, 751-755, 2001). In this synthesis system, genes of 31 factors including 3 initiation factors, 3 elongation factors, 4 factors involved in termination, 20 aminoacyl-tRNA synthetases that bind each amino acid to tRNA, and methionyl-tRNA formyl transferase, which constitute a protein synthesis system of bacteria, are amplified from Escherichia coli genome, and the protein synthesis system is reconstituted in vitro using these factors. In the present invention, such a reconstituted synthesis system may be used.

The term "cell-free transcription/translation system" is used interchangeably with a cell-free protein synthesis system, an in vitro translation system, or an in vitro transcription/translation system. In the in vitro translation system, a protein is synthesized using RNA as a template. As the template RNA, total RNA, mRNA, an in vitro transcript, or the like is used. In the other in vitro transcription/translation system, DNA is used as a template. The template DNA should contain a ribosome-binding region and preferably contains a suitable terminator sequence. In the in vitro transcription/translation system, conditions in which factors necessary for each reaction are added so that the transcription reaction and the translation reaction proceed continuously are set.

It is also possible to provide the transglutaminase obtained as described above by powdering the transglutaminase by, for example, freeze drying, vacuum drying, spray drying, or the like. At that time, the purified enzyme may be previously dissolved in an acetate buffer, a phosphate buffer, a triethanolamine buffer, a tris hydrochloride buffer, or a GOOD buffer. Preferably, an acetate buffer, a phosphate buffer, or a triethanolamine buffer can be used. Here, examples of the GOOD buffer include PIPES, MES, and MOPS.

### 2. Nucleic acid and transformant related to transglutaminase

A second aspect of the present invention provides a nucleic acid related to the transglutaminase.

Specifically, in one embodiment of the present invention, a DNA encoding an amino acid sequence of the transglutaminase is provided. The DNA encoding an amino acid sequence of the transglutaminase is typically used for preparation of the transglutaminase. According to a genetic engineering preparation method using the DNA encoding an amino acid sequence of the transglutaminase, it is possible to obtain the transglutaminase in a more homogeneous state. The method can also be said to be a suitable method when preparing a large amount of the transglutaminase. Applications of the DNA encoding an amino acid sequence of the transglutaminase are not limited to the preparation of the transglutaminase. For example, the DNA can also be used as an experimental tool for the purpose of elucidating a mechanism of action of the transglutaminase or the like, or as a tool for designing or producing a variant (modification) of the transglutaminase.

In the present invention, the DNA encoding an amino acid sequence of a transglutaminase refers to a nucleic acid from which the transglutaminase is obtained when it is expressed, and includes not only a nucleic acid having a base sequence corresponding to the amino acid sequence of the transglutaminase but also a nucleic acid obtained by adding a sequence not encoding an amino acid sequence to such a nucleic acid. Degeneracy of codons is also considered.

One embodiment of the DNA encoding an amino acid sequence of the transglutaminase is a DNA containing a base sequence set forth in SEQ ID NO: 4 or 5. The DNA of the base sequence set forth in SEQ ID NO: 4 encodes a full-length amino acid sequence (SEQ ID NO: 1) of a transglutaminase derived from Aspergillus oryzae. The DNA of SEQ ID NO: 5 encodes a partial amino acid sequence (SEQ ID NO: 2) excluding a localization sequence in a transglutaminase derived from Aspergillus oryzae.

In the present invention, as one embodiment of the DNA encoding an amino acid sequence of the transglutaminase, provided that a DNA having a base sequence that is partially different from the base sequence set forth in SEQ ID NO: 4 or 5 although a function of the transglutaminase encoded by the DNA is equivalent (hereinafter also referred to as "equivalent DNA". In addition, a base sequence that specifies the equivalent DNA is also referred to as "equivalent base sequence"). Examples of the equivalent DNA include a DNA containing a base sequence containing substitution, deletion, insertion, addition, or inversion of one or a plurality of bases with reference to the base sequence set forth in SEQ ID NO: 4 or 5, and encoding a protein having transglutaminase activity. Substitution, deletion, or the like of a base may occur at a plurality of sites. The term "plurality" as used herein is, for example, 2 to 40 bases, preferably 2 to 20 bases, and more preferably 2 to 10 bases, although it varies depending on a position and a type of an amino acid residue in a three-dimensional structure of a protein encoded by the DNA. The equivalent DNA has identity of, for example, 90% or more, preferably 92% or more, more preferably 94% or more, still more preferably 96% or more, still more preferably about 98% or more, and most preferably 99% or more to the base sequence set forth in SEQ ID NO: 4 or 5. The method for calculating the identity of a base sequence is as described in the section of "1. Transglutaminase" above.

The equivalent DNA as described above can be obtained, for example, by restriction enzyme treatment, treatment with exonuclease, DNA ligase, or the like, introduction of mutations by a site-directed mutagenesis method (Molecular Cloning, Third Edition, Chapter 13, Cold Spring Harbor Laboratory Press, New York) or a random mutagenesis method (Molecular Cloning, Third Edition, Chapter 13, Cold Spring Harbor Laboratory Press, New York), or the like. The equivalent nucleic acid can also be obtained by other methods such as ultraviolet irradiation.

Furthermore, in another embodiment of the present invention, a DNA encoding a putative amino acid sequence (SEQ ID NO: 3) of a transglutaminase active domain is also provided. Specific examples of the DNA include a DNA having a base sequence set forth in SEQ ID NO: 6; a DNA having identity of, for example, 90% or more, preferably 92% or more, more preferably 94% or more, still more preferably 96% or more, still more preferably about 98% or more, and most preferably 99% or more to an amino acid sequence set forth in SEQ ID NO: 6. The method for calculating the identity of a base sequence is as described in the section of "1. Transglutaminase" above.

Furthermore, in another embodiment of the present invention, there is provided a DNA having a base sequence complementary to a base sequence (e.g., a base sequence set forth in SEQ ID NO: 4 or 5) of a DNA encoding an amino acid sequence of the transglutaminase. Still another embodiment of the present invention provides a DNA having a base sequence that is at least about 90%, 92%, 94%, 96%, 98%, or 99% identical to a base sequence complementary to a base sequence (e.g., a base sequence set forth in SEQ ID NO: 4 or 5) of a DNA encoding an amino acid sequence of the transglutaminase. The method for calculating the identity of a base sequence is as described in the section of "1. Transglutaminase" above.

Still another embodiment of the present invention provides a DNA having a base sequence that hybridizes under stringent conditions to a base sequence complementary to a base sequence of a DNA encoding an amino acid sequence of the transglutaminase. The term "stringent conditions" as used herein refer to conditions under which a so-called specific hybrid is formed and a non-specific hybrid is not formed. Such stringent conditions are known to those skilled in the art, and can be set with reference to, for example, Molecular Cloning (Third Edition, Cold Spring Harbor Laboratory Press, New York) or Current protocols in molecular biology (edited by Frederick M. Ausubel et al., 1987). Specific examples of the stringent conditions include conditions of incubating at about 50°C using a hybridization solution (50% formamide, 10 × SSC (0.15 M NaCl, 15 mM sodium citrate, pH 7.0), 5 × Denhardt solution, 1% SDS, 10% dextran sulfate, 10 µg/ml denatured salmon sperm DNA, 50 mM phosphate buffer (pH 7.5)), and then washing at about 65°C using 0.1 × SSC, 0.1% SDS.

In still another embodiment of the present invention, there is provided a DNA that can be used as a probe for identifying a DNA encoding an amino acid sequence of the transglutaminase, and a DNA that can be used as a primer for, for example, amplifying or mutating a DNA encoding an amino acid sequence of the transglutaminase. The DNA used as such a probe or primer may be a DNA (DNA fragment) having part of a base sequence complementary to a base sequence of a DNA encoding an amino acid sequence of the transglutaminase. The DNA fragment is designed to include, for example, at least a portion that hybridizes to a contiguous nucleotide portion (e.g., about 10 to about 100 bases in length, preferably about 20 to about 100 bases in length, more preferably about 30 to about 100 bases in length) in a base sequence of a DNA encoding an amino acid sequence of the transglutaminase. When used as a probe, the DNA fragment can be labeled. For the labeling, for example, a fluorescent substance, an enzyme, or a radioactive isotope can be used.

Each of the above-described DNAs can be prepared in an isolated state by standard genetic engineering techniques, molecular biological techniques, biochemical techniques, chemical synthesis, PCR methods (e.g., overlap PCR), or combinations thereof with reference to sequence information disclosed in the present specification or the attached sequence listing.

Still another aspect of the present invention provides a vector containing a DNA encoding an amino acid sequence of the transglutaminase. As used herein, the term "vector" refers to a nucleic acid molecule capable of transporting a nucleic acid inserted therein into a target such as a cell.

An appropriate vector is selected depending on the intended use (cloning, protein expression) and in consideration of a type of a host cell. Examples of the vector using Escherichia coli as a host include M13 phage or a modification thereof, λ phage or a modification thereof, and pBR322 or a modification thereof (pB325, pAT153, pUC8, or the like). Examples of the vector using yeast as a host include pYepSec1, pMFa, and pYES2. Examples of the vector using an insect cell as a host include pAc and pVL. Examples of the vector using a mammalian cell as a host include pCDM8 and pMT2PC.

The vector of the present invention is preferably an expression vector. The term "expression vector" refers to a vector capable of introducing a nucleic acid inserted therein into a target cell (host cell) and expressing the nucleic acid in the cell. The expression vector usually contains a promoter sequence necessary for expression of the inserted nucleic acid, an enhancer sequence promoting expression, and the like. An expression vector containing a selection marker can also be used. When such an expression vector is used, the presence or absence (and degree) of introduction of the expression vector can be confirmed using a selection marker.

Insertion of DNA into a vector, insertion of a selection marker gene (if necessary), insertion of a promoter (if necessary), and the like can be performed using standard recombinant DNA techniques (e.g., known methods using restriction enzymes and DNA ligases, which can be with reference to Molecular Cloning, Third Edition, 1.84, Cold Spring Harbor Laboratory Press, New York).

Still another aspect of the present invention provides a transformant by transforming a host cell using the vector.

As the host cell, microorganisms such as koji mold (Aspergillus oryzae), Escherichia coli, Bacillus subtilis, and Saccharomyces cerevisiae are preferably used from the viewpoint of ease of handling, but any host cell capable of replicating recombinant DNA and expressing a gene of the present enzyme can be used. Examples of the Escherichia coli include Escherichia coli BL21 (DE3) pLysS when a T7 promoter is used, and Escherichia coli JM109 otherwise. Examples of the Saccharomyces cerevisiae include Saccharomyces cerevisiae SHY2, Saccharomyces cerevisiae AH22, and Saccharomyces cerevisiae INVSc1 (Invitrogen).

The transformant can be obtained by transfection or transformation. For example, the method can be performed by a calcium chloride method (Journal of Molecular Biology (J. Mol. Biol.), vol. 53, p. 159 (1970)), a Hanahan method (Journal of Molecular Biology, vol. 166, p. 557 (1983)), an SEM method (Gene, vol. 96, p. 23 (1990)], a method of Chung et al. (Proceedings of the National Academy of Sciences of the United States of America, vol. 86, p. 2172 (1989)), a calcium phosphate coprecipitation method, electroporation (Potter, H. et al., Proc. Natl. Acad. Sci. U.S.A. 81, 7161-7165 (1984)), lipofection (Felgner, P.L. et al., Proc. Natl. Acad. Sci. U.S.A. 84, 7413-7417 (1984)), or the like. The transformant can be used for producing the present enzyme of the present invention.

### 3. Enzyme preparation

Still another aspect of the present invention provides an enzyme preparation containing the transglutaminase as an active ingredient. As used herein, the term "enzyme preparation containing a transglutaminase as an active ingredient" is a preparation that contains the transglutaminase and is used for the purpose of expressing transglutaminase activity (i.e., a function of catalyzing an acyl transfer reaction of a γ-carboxylamide group of a glutamine residue in a peptide chain).

The degree of purification of the transglutaminase in the enzyme preparation is not particularly limited. The final form of the enzyme preparation may be a liquid form or a solid form (including a powder form).

The content of an active ingredient (the transglutaminase) in the enzyme preparation is not particularly limited, but, for example, the content of the active ingredient can be set or adjusted so that transglutaminase activity as measured by the transglutaminase activity measurement method described above is 0.01 U to 10,000 U, preferably 0.1 U to 1000 U per 1 g of the enzyme preparation. The enzyme preparation is usually provided in a solid form (e.g., an immobilized enzyme in which the enzyme is immobilized on a material capable of immobilizing the enzyme on the surface or inside of a granule, a powder, silica, a porous polymer, or the like) or in a liquid form. The enzyme preparation may contain, in addition to an active ingredient (the transglutaminase), an excipient, a buffer, a suspending agent, a stabilizer, a preservative, an antiseptic, physiological saline, or the like. As the excipient, lactose, sorbitol, D-mannitol, maltodextrin, saccharose, or the like can be used. As the buffer, phosphate, citrate, acetate, or the like can be used. As the stabilizer, propylene glycol, ascorbic acid, or the like can be used. As the preservative, phenol, benzalkonium chloride, benzyl alcohol, chlorobutanol, methylparaben, or the like can be used. As the antiseptic, benzalkonium chloride, paraoxybenzoic acid, chlorobutanol, or the like can be used.

### 4. Applications of transglutaminase and enzyme preparation

A further aspect of the present invention provides applications of the transglutaminase and the enzyme preparation. Specifically, the transglutaminase and the enzyme preparation are used in an application for catalyzing an acyl transfer reaction of a γ-carboxylamide group of a glutamine residue in a protein or a peptide. When the transglutaminase and the enzyme preparation are allowed to act on a protein or a peptide, usually, an ε-amino group of a lysine residue and a γ-carboxylamide group of a glutamine residue in the protein or the peptide are condensed to form an ε-(γ-Gln)-Lys crosslinking bond in a molecule or between molecules of the protein or the peptide, and a crosslinked structure is formed in the molecule or between the molecules. Therefore, the transglutaminase and the enzyme preparation can be used for the purpose of modifying a protein or a peptide by crosslinking the protein or the peptide.

As used herein, with respect to a substrate for the transglutaminase and the enzyme preparation, the term "protein" refers to a compound in which 50 or more amino acids are peptide-linked, and the term "peptide" refers to a compound in which less than 50 amino acids are peptide-linked. The peptide as a substrate for the transglutaminase and the enzyme preparation desirably contains at least one glutamine residue and at least one lysine residue.

When the transglutaminase or the enzyme preparation is allowed to act on a protein or a peptide, for example, the transglutaminase or the enzyme preparation may be added so as to have a transglutaminase activity value of 1000 to 0.0001 U, preferably 100 to 0.001 U, more preferably 50 to 0.01 U per 1 g of the protein or the peptide.

In addition, a reaction temperature when the transglutaminase or the enzyme preparation is allowed to act on a protein or a peptide may be appropriately set according to an amount of the transglutaminase or the enzyme preparation added, a degree of modification of the protein or the peptide, and the like, and the reaction temperature is, for example, 5 to 70°C, preferably 10 to 60°C, and more preferably 20 to 50°C.

In addition, a reaction time when the transglutaminase or the enzyme preparation is allowed to act on a protein or a peptide may be appropriately set according to an amount of the transglutaminase or the enzyme preparation added, a degree of modification of the protein or the peptide, and the like, and the reaction time is, for example, 5 minutes to 24 hours, preferably 10 minutes to 12 hours, and more preferably 15 minutes to 6 hours.

The transglutaminase and the enzyme preparation can be used for, for example, modification of proteins or peptides used as food materials; modification of proteins or peptides used in medicine; and modification of proteins or peptides used in industrial products such as fibers, leather, and immobilized enzymes. In other words, as examples of applications of the transglutaminase and the enzyme preparation, a method for producing a food (e.g., processed meat products such as sausages and molded meat, processed soybean products such as bean curds, processed wheat products such as bread and noodles, processed marine products such as fish paste, and processed milk products such as yogurt), medical applications (antibody drug conjugate or regenerative medical applications), and industrial applications (production of fibers, leather, and immobilized enzymes) are provided. In the method for producing a food of the present invention, the present enzyme preparation is allowed to act on a food material (for example, the present enzyme preparation is added to form a state in which the enzyme as an active ingredient acts) to cause an enzymatic reaction. In medical applications, the present enzyme preparation is allowed to act on a pharmaceutical material, or the enzyme preparation itself is used for medical applications to cause an enzymatic reaction. In industrial applications, the present enzyme preparation is allowed to act on an industrial material to cause an enzymatic reaction.

### EXAMPLE

### <Search for protein having transglutaminase activity>

From 776 genes with unknown functions present in Aspergillus oryzae, 60 genes localized in a septal pore were found. Among them, disrupted strains were obtained for specific genes, and a hypotonic shock experiment was performed. Whether or not it was possible to prevent lysis of hyphal tip cells from propagating to adjacent cells was confirmed, and a protein having transglutaminase (TGase) activity was searched for.

The method of the hypotonic shock experiment used for evaluation of transglutaminase activity is shown below.

### (Hypotonic shock experiment)

Lysis of hyphal tips was induced by adding 1 ml of water to Aspergillus oryzae colonies and grown on a thin DPY agar medium (0.5% yeast extract, 1% Hipolypeptone, 2% dextrin, 0.5% KH₂PO₄, 0.05% MgSO₄·7H₂O, 1.5% agar) of a glass bottom dish at 30°C for 18 hours. Thirty hyphae showing lysis of hyphal tips were randomly selected and observed with a differential interference contrast microscope. If cytoplasm of cells adjacent to the lysed tip cells was retained and propagation of lysis could be prevented, it was determined that the protein had transglutaminase activity (Fig. 1).

### 1. Production of gene-disrupted strains

### 1-1. Construction and evaluation of septum-localized protein gene-disrupted strains

An upstream region of 1.5 kb and a downstream region of 1.5 kb and a pyrG marker of each gene of 60 septum-localized proteins were amplified from the genomic DNA of an Aspergillus oryzae RIB40 strain. Three amplified DNA fragments (upstream region, downstream region, pyrG marker of each gene) and a pUC19 vector were fused using In-Fusion HD Cloning Kit (Clontech Laboratories, Inc., Mountain View, CA, USA). Using the obtained plasmid as a template, a DNA fragment for gene disruption was amplified. The amplified PCR fragment was precipitated with ethanol and introduced into the corresponding locus of an Aspergillus oryzae NSP1D1 strain (niaD- sC- ΔpyrG ΔligD) by homologous recombination. The resulting disrupted strain of each gene and a wild-type strain (NSP1D1 strain) were subjected to a hypotonic shock experiment. As a result, it was found that ability to prevent propagation of lysis was the lowest in a disrupted strain of an AO090023000250 gene (hereinafter abbreviated as "AO090023000250") containing a sequence of SEQ ID NO: 4 among the genes of 60 septum-localized proteins (Fig. 2), and it was inferred that AO090023000250 had transglutaminase activity. The amino acid sequence encoded by AO090023000250 is set forth in SEQ ID NO: 1.

### 1-2. Construction and evaluation of strains in which amino acids at active center are substituted of AO090023000250

Next, the presence or absence of the ability to prevent propagation of lysis when three residues (Cys417, His464, Asp480) presumed to constitute a transglutaminase active center of AO090023000250 were substituted with alanine was confirmed. First, AO090023000250 was amplified by PCR using genomic DNA of an Aspergillus oryzae RIB40 strain as a template and using a primer designed so that an alanine substitution is introduced into Cys417, His464, or Asp480. The resulting DNA fragment was fused with a pUXN vector cleaved with SmaI (Mori et al. BiosciBiotechnol Biochem 83: 1557-1569, 2019). The plasmid into which each mutant gene was introduced was isolated, digested with NotI, and then introduced into a niaD locus of the AO090023000250-disrupted strain by homologous recombination. A disrupted strain into which each mutant gene was introduced, the AO090023000250-disrupted strain, and a wild-type strain (NSP1D1 strain) were subjected to a hypotonic shock experiment. As a result, in Cys417Ala and His464Ala-substituted strains, the ability to prevent propagation of lysis was reduced to the same level as that of the gene-deleted strains. In addition, even with amino acid substitution of Asp480Ala, a decrease in the ability to prevent propagation of lysis in hyphal damage was observed (Fig. 3). The above results support the estimation that "Cys417, His464, and Asp480 constitute a transglutaminase active center".

### 1-3. Construction and evaluation of partially deleted strains of AO090023000250

Partial deletants of a putative transglutaminase protein of A. oryzae (having an amino acid sequence set forth in SEQ ID NO: 1) were produced. First, a DNA fragment encoding an N-terminus-deleted form (SEQ ID NO: 2; a region from positions 359 to 697 of the amino acid sequence set forth in SEQ ID NO: 1) was amplified using a primer designed so that an N-terminal region (region from positions 1 to 358 of the amino acid sequence set forth in SEQ ID NO: 1) is deleted. As a template, genomic DNA of an Aspergillus oryzae RIB40 strain was used. Similarly, a DNA fragment encoding a C-terminus-deleted form (region from positions 1 to 358 of the amino acid sequence set forth in SEQ ID NO: 1) designed so that a C-terminal region (region from positions 359 to 697 of the amino acid sequence set forth in SEQ ID NO: 1) is deleted and a DNA fragment encoding an N-terminus/C-terminus-deleted form (region from positions 359 to 481 of the amino acid sequence set forth in SEQ ID NO: 1) designed so that a C-terminal region (region from positions 482 to 697 of the amino acid sequence set forth in SEQ ID NO: 1) and an N-terminal region (regions 1 to 358 of the amino acid sequence set forth in SEQ ID NO: 1) are deleted were each amplified. Each of the three DNA fragments prepared by the above method was fused with a plasmid designed so that EGFP (green fluorescent protein) is fused to the C-terminus. A DNA obtained by digesting each plasmid with NotI, followed by ethanol precipitation, was introduced into a niaD locus of the AO090023000250-disrupted strain by homologous recombination. Lysis was induced by a hypotonic shock experiment, and as a result of observation with a fluorescence microscope, the C-terminus-deleted form was accumulated in a septal pore during lysis, whereas the N-terminus-deleted form and the N-terminus/C-terminus-deleted form were uniformly localized in the cytoplasm (Fig. 4). From the above results, it was found that the N-terminal region (region at positions 1 to 358 of the amino acid sequence set forth in SEQ ID NO: 1) was involved in accumulation in a septal pore in hyphal damage, in other words, the N-terminal region was a region involved in not a transglutaminase activity but localization of an enzyme. An amino acid sequence of a putative transglutaminase active domain (region from positions 359 to 481 of the amino acid sequence set forth in SEQ ID NO: 1) is set forth in SEQ ID NO: 3.

### 2. Preparation and evaluation of transglutaminase

### 2-1. Preparation of transglutaminase

A putative TGase protein of A. oryzae was expressed and purified as a 3 × HA-6His tag fusion protein, and transglutaminase activity was measured using polymerization of casein as an index. ORF of AO090023000250 was amplified with a primer designed so that a 3 × HA-6His tag is fused to the C-terminal side using genomic DNA of RIB40 as a template. The amplified DNA fragment was fused with a pUt-NAN vector cleaved with SmaI (Katayama et al. Appl Environ Microbiol 85: 1-16, 2019) using In-Fusion HD Cloning Kit. The produced plasmid was cleaved with NotI and introduced into a niaD locus of an NSlDl strain by homologous recombination.

A strain expressing the 3 × HA-6His tag fusion protein was inoculated into a DPY liquid medium (0.5% yeast extract, 1% Hipolypeptone, 2% dextrin, 0.5% KH₂PO₄, 0.05% MgSO₄·7H₂O) and cultured at 30°C for 18 hours. The cell body after culture was collected, frozen with liquid nitrogen, and disrupted. An extraction buffer (50 mM Tris-HCl (pH 8.0), 200 mM NaCl, 1 mM PMSF) was added to suspend the cell body-disrupted powder. The suspension was held on ice for 10 minutes, and then centrifuged to obtain a supernatant. The 3 × HA-6His tag fusion protein was purified using Ni-NTA Purification System (Thermo Fisher Scientific, Inc., Waltham, MA), and the fraction eluted with imidazole was dialyzed and concentrated by ultrafiltration centrifugation.

### 2-2. Evaluation of transglutaminase

5 to 10 µL of an enzyme solution (purified or unpurified 3 × HA-6His tag fusion protein solution) was mixed with 10 µL of a 10% N,N-dimethylated casein solution and 35 µL of a reaction buffer (50 mM HEPES (pH 7.5), 500 mM NaCl, 10 mM CaCl2, 1 mM DTT), and the mixture was reacted at 30°C for 2 hours. The sample after the reaction was migrated by SDS-PAGE, and polymerization of casein was analyzed by Coomassie blue staining. Fig. 5 shows that polymerization of casein occurred in the presence of the purified or unpurified 3 × HA-6His tag fusion protein. In the absence of the purified or unpurified 3 × HA-6His tag fusion protein, a band of monomeric casein was observed around about 30 kDa, whereas in the presence of the purified or unpurified 3 × HA-6His tag fusion protein, polymerization of casein was observed. From the above results, it was suggested that AO090023000250 had transglutaminase activity.

### 3. Observation of transglutaminase activity using 5-FITC cadaverine

5-FITC cadaverine is a fluorescent substrate for detecting transglutaminase activity. Since cadaverine is labeled with a fluorescent substance FITC, a crosslinked product can be detected without using another fluorescent probe. A specific experimental method is as follows.

On a 50 µl DPY agar medium thinly spread on a glass bottom dish, conidia of the AO090023000250-disrupted strain produced above and a wild-type strain (NSP1D1 strain) were inoculated and cultured for 16 hours. Colonies were treated with 50 µl of a 20 µM 5-FITC cadaverine (AnaSpec Inc., USA) solution at room temperature with gentle shaking for 15 minutes. The colonies were then washed with 1 ml of sterile water and the sterile water was removed. Thereafter, a hypotonic shock experiment was performed, and fluorescence in a septal pore adjacent to lysed hyphal tip cells was observed with a fluorescence microscope. For only the hyphae that prevented propagation of lysis in each strain, a proportion of hyphae in which fluorescence was observed in a septal pore was determined. Ten hyphae were analyzed in each experiment, and three independent experiments were performed to calculate an average value of the proportion of hyphae in which fluorescence was observed in a septal pore.

Green fluorescence showing transglutaminase activity was observed in the septal pore adjacent to hyphal tip cells lysed by a hypotonic shock. In the wild-type strain and the AO090023000250-disrupted strain, the presence or absence of transglutaminase activity in a septal pore was counted only for the hyphae that prevented propagation of lysis. As a result, in the AO090023000250-disrupted strain, a decrease in septal pores showing fluorescence could be confirmed (Fig. 6). From the above results, it was confirmed that AO090023000250 encoded a protein having transglutaminase activity.

### INDUSTRIAL APPLICABILITY

The novel transglutaminase of the present invention is derived from koji mold and is also suitable for food applications. Further, the present invention is applicable not only to foods but also to various applications. Thus, the applications of the present invention are wide and their industrial utility values are extremely high.

The present invention is not limited to the description of the embodiments and examples of the invention above. Various modifications that can be easily conceived by those skilled in the art without departing from the description of the claims are also included in the present invention. The entire contents of the articles, published patent publications, patent publications, and the like specified in the present specification are incorporated herein by reference.

## Claims

1. A transglutaminase consisting of a protein represented by any one of (a) to (c):
(a) a protein containing an amino acid sequence set forth in SEQ ID NO: 1 or 2;
(b) a protein containing an amino acid sequence in which one or a few amino acids are substituted, added, inserted, or deleted in an amino acid sequence set forth in SEQ ID NO: 1 or 2, and having activity of catalyzing an acyl transfer reaction of a γ-carboxylamide group of a glutamine residue in a peptide chain; and
(c) a protein containing an amino acid sequence having sequence identity of 90% or more to an amino acid sequence set forth in SEQ ID NO: 1 or 2, and having activity of catalyzing an acyl transfer reaction of a γ-carboxylamide group of a glutamine residue in a peptide chain.

2. The transglutaminase according to claim 1, which is derived from Aspergillus oryzae.

3. A DNA encoding the transglutaminase according to claim 1 or 2.

4. The DNA according to claim 3, comprising a base sequence having sequence identity of 90% or more to a base sequence of SEQ ID NO: 4.

5. An enzyme preparation comprising the transglutaminase according to claim 1 or 2 as an active ingredient.

6. The enzyme preparation according to claim 5, which is used for crosslinking a protein or a peptide.

7. The enzyme preparation according to claim 5, which is for production of a food.

8. The enzyme preparation according to claim 5, which is for medical use or industrial use.

9. A method for performing an acyl transfer reaction on a γ-carboxylamide group of a glutamine residue in a protein or a peptide, the method comprising allowing a protein represented by any one of (a) to (c) to act on a protein or a peptide as a substrate:
(a) a protein containing an amino acid sequence set forth in SEQ ID NO: 1 or 2;
(b) a protein containing an amino acid sequence in which one or a few amino acids are substituted, added, inserted, or deleted in an amino acid sequence set forth in SEQ ID NO: 1 or 2, and having activity of catalyzing an acyl transfer reaction of a γ-carboxylamide group of a glutamine residue in a peptide chain; and
(c) a protein containing an amino acid sequence having sequence identity of 90% or more to an amino acid sequence set forth in SEQ ID NO: 1 or 2, and having activity of catalyzing an acyl transfer reaction of a γ-carboxylamide group of a glutamine residue in a peptide chain.

10. The acyl transfer method according to claim 9, which is performed for crosslinking a protein or a peptide.

11. A method for producing a food, the method comprising allowing a protein represented by any one of (a) to (c) to act on a food material containing a protein or a peptide:
(a) a protein containing an amino acid sequence set forth in SEQ ID NO: 1 or 2;
(b) a protein containing an amino acid sequence in which one or a few amino acids are substituted, added, inserted, or deleted in an amino acid sequence set forth in SEQ ID NO: 1 or 2, and having activity of catalyzing an acyl transfer reaction of a γ-carboxylamide group of a glutamine residue in a peptide chain; and
(c) a protein containing an amino acid sequence having sequence identity of 90% or more to an amino acid sequence set forth in SEQ ID NO: 1 or 2, and having activity of catalyzing an acyl transfer reaction of a γ-carboxylamide group of a glutamine residue in a peptide chain.

12. Use of a protein represented by any one of (a) to (c) for performing an acyl transfer reaction on a γ-carboxylamide group of a glutamine residue in a protein or a peptide:
(a) a protein containing an amino acid sequence set forth in SEQ ID NO: 1 or 2;
(b) a protein containing an amino acid sequence in which one or a few amino acids are substituted, added, inserted, or deleted in an amino acid sequence set forth in SEQ ID NO: 1 or 2, and having activity of catalyzing an acyl transfer reaction of a γ-carboxylamide group of a glutamine residue in a peptide chain; and
(c) a protein containing an amino acid sequence having sequence identity of 90% or more to an amino acid sequence set forth in SEQ ID NO: 1 or 2, and having activity of catalyzing an acyl transfer reaction of a γ-carboxylamide group of a glutamine residue in a peptide chain.

13. The use according to claim 12, which is for crosslinking a protein or a peptide.
